# EUROPÄISCHE PATENTSCHRIFT

(11) **EP 0 745 360 B1**
(45) Veröffentlichungstag und Bekanntmachung des Hinweises auf die Patenterteilung: **17.10.2001**
(21) Anmeldenummer: 96107855.7
(22) Anmeldetag: 17.05.1996
(51) Int. Cl.: A61F 2/34

(54) **Künstliche Hüftgelenkpfanne**
Artificial acetabular cup
Cotyle artificiel de la hanche

(30) Priorität: 03.06.1995 DE 19520468
(43) Veröffentlichungstag der Anmeldung: 04.12.1996
(73) Patentinhaber: Scholz, Werner, 30173 Hannover (DE)
(72) Erfinder: Scholz, Werner, 30173 Hannover (DE)
(74) Vertreter: Leine, Sigurd, Dipl.-Ing.

(56) Entgegenhaltungen:
- DE-A- 2 645 101
- DE-A- 3 101 333
- DE-U- 9 402 941
- FR-A- 2 630 907
- US-A- 4 662 891

## Beschreibung

Die Erfindung betrifft eine künstliche Hüftgelenkpfanne der im Oberbegriff des Anspruchs 1 genannten Art.

Durch DE 36 02 081 A1 ist eine künstliche Hüftgelenkpfanne bekannt, bei der die äußere Kontur des selbstschneidenden Gewindes aus mehreren Konen besteht, wobei das Gewinde ein dreieckiges Profil mit einem breiten Gewindegrund hat. Die konische äußere Form des Gewindes soll zur Erleichterung des Einschraubvorganges dienen. Eine bündige Anlage des Kerns des Gewindes wird nicht erreicht. Vielmehr ist in der Hüftgelenkpfanne eine Öffnung vorgesehen, die dazu dient, daß der Operateur nach dem Einsetzen der Hüftpfanne in das Becken die Möglichkeit hat, Hohlräume zwischen der Außenfläche der Hüftgelenkpfanne und dem Knochen mit Knochenspänen auszufüllen. Die Hüftgelenkpfanne ist somit nicht sofort voll belastbar, sondern erst dann, wenn die Knochenspäne sich verwachsen und verfestigt haben.

Durch EP 0 259 420 B1 ist eine künstliche Hüftgelenkpfanne bekannt, bei der die gesamte Außenfläche teilkugelförmig ist, also auch im Bereich des selbstschneidenden Außengewindes. Dieses ist trapezförmig, wobei die Außenfläche seines Kernes eine Verlängerung des vorderen, teilkugelförmigen Abschnittes bildet. Die durchlaufende Kugelform soll ein Einschrauben in eine komplementär kugelig ausgebildete Ausnehmung im Hüftknochen eines Patienten in verschiedenen Richtun gen ermöglichen, um so die Lage der Hüftgelenkpfanne im eingeschraubten Zustand der jeweiligen anatomischen Situation anzupassen.

Ein Nachteil dieser bekannten Gelenkpfanne besteht darin, daß es schwierig ist, den Grund des trapezförmigen Gewindes der Kugelform anzupassen; denn während des Schneidens des Gewindes mittels einer Drehmaschine ändert sich nicht nur die radiale Lage des Gewindegrundes, sondern auch dessen Ausrichtung.

Nach DE 40 31 926 A1 soll die Kugelform im Gewindegrund dadurch hergestellt werden, daß das Spanen mit mehreren Werkzeugen unterschiedlicher Schnittkantengeometrie erfolgt. Durch die Verwendung mehrerer Werkzeuge ist der Aufwand beträchtlich. Außerdem ist nur eine abschnittsweise Anpassung möglich. Eine genaue Anpassung wäre nur durch die Verwendung einer unendlichen Zahl von Werkzeugen möglich, die nacheinander an den aufeinanderfolgenden Stellen des Gewindes zum Einsatz kommen müßten.

Durch EP 0 482 320 A1 ist eine Hüftgelenkendoprothese bekannt, an deren Außenfläche sich ein selbstschneidendes, innen trapezförmig, außen jedoch spitzes Gewinde befindet. Die Außenfläche und damit auch der Gewindekern ist kegelstumpfförmig. Durch die Kegelstumpfform des Gewindekernes hat der Gewindegrund immer die gleiche Ausrichtung, so daß zum Schneiden des Gewindes mittels einer Drehmaschine nur ein Schneidwerkzeug einer vorbestimmten Schnittkantengeometrie erforderlich ist. Ein entscheidender Nachteil dieser bekannten Hüftgelenkendoprothese besteht jedoch darin, daß ein Einschrauben in eine komplementär geformte Ausnehmung im Hüftgelenkknochen nur in einer mit der Ausnehmung koaxialen Richtung möglich ist.

Durch DE-U-94 02 941 ist eine künstliche Hüftgelenkpfanne der im Oberbegriff des Anspruchs 1 genannten Art bekannt, bei der der Kern des trapezförmigen Außengewindes mit seiner Oberfläche im gesamten Bereich des Außengewindes die als Verlängerung des teilkugelförmigen Abschnitts der Hüftgelenkpfanne gedachte Kugelfläche an allen Stellen unterragt mit der Folge, daß der Kern des Außengewindes im eingeschraubten Zustand an der Innenwandung der kugelförmigen Ausfräsung im Hüftknochen nicht anliegt und damit keine Kräfte übertragen kann. Da das Außengewinde lediglich den Zweck hat, das Implantat in der Ausfräsung des Hüftknochens zu halten und entsprechend ausgebildet ist, kann es nur in unwesentlichem Maße zur Übertragung von Kräften beitragen. Die Kraftübertragung im Bereich des Außengewindes ist somit schlecht.

Der Erfindung liegt die Aufgabe zugrunde, eine künstliche Hüftgelenkpfanne der im Oberbegriff des Anspruchs 1 genannten Art zu schaffen, die einfach herzustellen ist, die ein Einschrauben in eine kugelförmige Ausnehmung im Hüftknochen in unterschiedliche Richtungen erlaubt, während gleichzeitig ein bündiges Einschrauben gewährleistet ist.

Die der Erfindung zugrundeliegende Aufgabe wird durch die im Kennzeichen des Anspruchs 1 angegebene Lehre gelöst.

Der Grundgedanke dieser Lehre besteht darin, die wesentlichen tragenden Teile der Außenfläche kugelig auszubilden, damit nach dem Einschrauben eine bündige Anlage an der Innenfläche einer komplementär kugelig geformten Ausnehmung im Hüftknochen erfolgt. Der das Außengewinde aufweisende hintere Abschnitt der Hüftgelenkpfanne ist dagegen kegelig und bildet den Grund des Außengewindes, wonach sich zunächst der Vorteil einer einfachen und exakten Herstellung des selbstschneidenden Außengewindes ergibt.

Gemäß der Erfindung überragt der das selbstschneidende Gewinde tragende kegelige Kern wenigstens teilweise die verlängert gedachte Kugelfläche nach außen. Dadurch würde an sich ein Klemmen beim Einschrauben auftreten. Dies wird erfindungsgemäß jedoch dadurch vermieden, daß die kegelige Außenfläche des Gewindekerns selbstschneidenden Charakter hat, so daß ein Klemmen nicht mehr eintreten kann.

Die Schneidkanten an der freien Außenfläche des Gewindekerns fräsen beim Einschrauben die Wandung in der kugelförmigen Ausnehmung des Hüftknochens komplementär aus, so daß zwangsläufig beim Einschrauben eine bündige Anlage erreicht wird, obwohl zunächst die sich entsprechenden Flächenteile nicht komplementär sind. Dadurch liegt im implantierten Zustand die Außenfläche der Hüftgelenkpfanne nicht nur in ihrem vorderen, teilkugelförmigen Bereich an, sondern auch im Bereich des Gewindegrundes, so daß sich insgesamt eine geringere spezifische Belastung des Knochengewebes im Bereich der Oberfläche der Ausfräsung im Hüftknochen und damit auch eine größere Dauerhaftigkeit der Hüftgelenkpfanne ergibt.

Die Schneidkanten können an Vorsprüngen oder Vertiefungen an der freien Außenfläche des Kerns des Gewindes gebildet sein. Im Falle von Vertiefungen entspricht die selbstschneidende Wirkung der bei dem selbstschneidenden Gewinde. Im Falle von Vorsprüngen wird mehr Knochen aus der kugelförmigen Wandung der Ausnehmung im Hüftknochen ausgefräst. Es ist auch möglich, Vorsprünge und Vertiefungen gleichzeitig vorzusehen. Das hängt alles von den jeweiligen Gegebenheiten, insbesondere der Beschaffenheit des Hüftknochens, ab.

Das selbstschneidende Gewinde kann in an sich bekannter Weise nach außen hin spitz sein.

Gemäß einer zweckmäßigen Weiterbildung der Erfindung überragt der Kern des selbstschneidenden Gewindes die verlängert gedachte Kugelfläche am Anfang und am Ende des Gewindes nach außen, während er sie im mittleren Bereich unterragt. Bei dieser Ausführungsform erfolgt ein komplementäres Ausfräsen nur am Anfang und am Ende des Gewindes. In dessen mittlerem Bereich verbleibt dagegen ein kleiner freier Raum zwischen Gewindekern und Wandung der Ausnehmung im Hüftknochen. In diesen Raum kann sich das am Anfang und am Ende des Gewindes ausgefräste Knochenmaterial einlagern.

Obwohl gemäß der Lehre der Erfindung der hintere, das selbstschneidende Außengewinde aufweisende Abschnitt der Außenfläche der Hüftgelenkpfanne grundsätzlich kegelig ist, können doch die Spitzen des Gewindes in einer Kugelfläche liegen, die konzentrisch zu der teilkugelförmigen Außenfläche ist. Dadurch kann das Einschrauben der Hüftgelenkpfanne während der Anfangsphase erleichtert werden.

Anhand der Zeichnung soll die Erfindung an einem Ausführungsbeispiel näher erläutert werden.
- Fig. 1: zeigt ein Ausführungsbeispiel der erfindungsgemäßen Hüftgelenkpfanne in der Draufsicht und
- Fig. 2: ist ein Schnitt II - II durch Fig. 1.

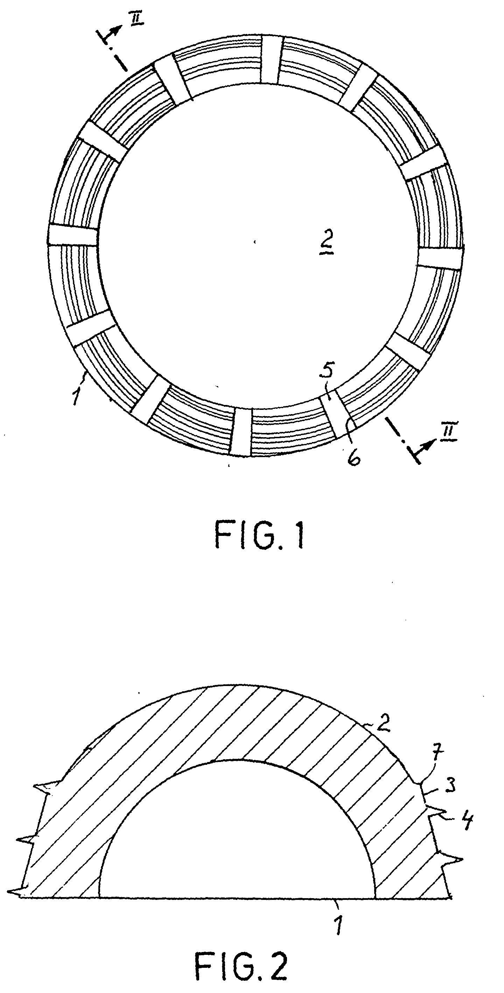

Die Fig. 1 und 2 zeigen eine künstliche Hüftgelenkpfanne 1, deren Außenfläche einen vorderen, teilkugelförmigen Abschnitt 2 und einen hinteren, kegelförmigen Abschnitt 3 aufweist, von dem aus sich ein selbstschneidendes Gewinde 4 erstreckt, das nach außen spitz und nach innen trapezförmig ist, wobei der Gewindegrund durch den kegelförmigen Abschnitt 3 gebildet ist.

Aus Fig. 1 ist zu ersehen, daß der selbstschneidende Charakter des Gewindes 4 dadurch erzeugt ist, daß die Gewindegänge 4 durch Vertiefungen 5 mehrfach unterbrochen sind, so daß scharfe Kanten 6 gebildet sind. Diese scharfen Kanten erstrecken sich über den gesamten Bereich des kegelförmigen Abschnittes 3 bis zu einer inneren Kante 7, an der der kegelförmige Abschnitt endet und der kugelförmige Abschnitt 2 beginnt.

Aus Fig. 2 ist zu ersehen, daß der Kern des selbstschneidenden Gewindes, also die Außenfläche des kegelförmigen Abschnittes 3, die verlängert gedachte Fläche des teilkugelförmigen Abschnittes 2 am Anfang und am Ende des Gewindes nach außen überragt, im mittleren Bereich dagegen unterragt.

Wird die künstliche Hüftgelenkpfanne gemäß den Fig. 1 und 2 in eine kugelförmige, also mittels eines Kugelfräsers ausgearbeitete Ausnehmung in einem Hüftgelenkknochen eingeschraubt, so kommen zunächst die Spitzen des selbstschneidenden Gewindes 4 an der Innenwandung der Ausnehmung zur Anlage, wonach durch die selbstschneidende Wirkung das Gewinde 4 sich selbst ein Innengewinde schneidet. Ganz zum Schluß des Einschraubvorganges, also kurz bevor der kugelförmige Abschnitt 2 der Außenfläche der Hüftgelenkpfanne bündig zur Anlage kommt, kommen die Schneidkanten 6 im Bereich des Gewindegrundes zur Wirkung, so daß in der Ausnehmung im Hüftknochen eine komplementär kegelige Innenfläche erzeugt wird, so daß zum Schluß des Einschraubvorganges, wenn der kugelige Abschnitt 2 bündig zur Anlage kommt, auch der hintere kegelförmige Abschnitt 7 bündig an seiner von ihm selbst geschaffenen Innenfläche im Knochen zur Anlage kommt. Trotz der einfachen und einfach herzustellenden Form des Gewindes 4 und insbesondere des Gewindegrundes ergibt sich somit eine bündige Anlage der Hüftgelenkpfanne im gesamten Bereich der Außenfläche.

## Patentansprüche

1. Künstliche Hüftgelenkpfanne, mit einer räumlich gekrümmten Außenfläche, die einen vorderen, teilkugelförmigen Abschnitt (2) und einen hinteren, ein selbstschneidendes, sich von einem kegelförmigen Kern aus erstreckendes Außengewinde (4) aufweisenden Abschnitt (3) aufweist, wobei das Außengewinde (4) die Kugelfläche nach außen überragt, die als Verlängerung des teilkugelförmigen Abschnittes gedacht ist, und wobei die Flanken des Außengewindes (4) im Bereich des Kerns einen Abstand voneinander haben, **dadurch gekennzeichnet, daß** der das selbstschneidende Außengewinde (4) tragende Kegelförmige Kern die als Verlängerung des teilkugelförmigen Abschnittes gedachte Kugelfläche wenigstens teilweise nach außen überragt, daß an der Außenfläche des Gewindekerns Schneidkanten (6) gebildet sind und daß sich die Ausnehmungen (5), die die Schneidkanten (6) des selbstschneidenden Außengewindes (4) bilden, wenigstens bis zu der verlängert gedachten Kugelfläche erstrecken.

2. Künstliche Hüftgelenkpfanne nach Anspruch 1, **dadurch gekennzeichnet, daß** die Schneidkanten (6) an Vorsprüngen an der freien Außenfläche des Kerns gebildet sind.

3. Künstliche Hüftgelenkpfanne nach Anspruch 1, **dadurch gekennzeichnet, daß** das selbstschneidende Außengewinde (4) nach außen hin spitz ist.

4. Künstliche Hüftgelenkpfanne nach Anspruch 1, **dadurch gekennzeichnet, daß** der Kern des selbstschneidenden Außengewindes (4) die verlängert gedachte Kugelfläche am Anfang und am Ende des Außengewindes (4) nach außen überragt, im mittleren Bereich unterragt.

5. Künstliche Hüftgelenkpfanne nach Anspruch 4, **dadurch gekennzeichnet, daß** die Spitzen des Außengewindes (4) in einer Kugelfläche liegen, die konzentrisch zu dem teilkugelförmigen Abschnitt (2) ist.

## Claims

1. An artificial acetabular cup with a spatially curved outer surface, having a front part-spherical shaped section (2) and a rear section (3) having a self-cutting outer thread (4) extending from a conical-shaped core, where the outer thread (4) projects outwardly over the spherical surface, which is intended as an extension of the part-spherical section, and where the flanks of outer thread (4) are spaced from one another in the area of the core **characterised in that** the self-cutting thread (4) bearing the conical-shaped core projects at least partially out over the spherical surface, intended as an extension of the part-spherical shaped section, that cutting edges (6) are constructed on the outer surface of the threaded core, and that recesses (5) which form the cutting edges (6) of self-cutting thread (4), reach at least as far as the intended extended spherical surface.

2. An artificial acetabular cup according to claim 1, **characterised in that** cutting edges (6) are constructed on projections on the free outer surface of the core.

3. An artificial acetabular cup according to claim 1, **characterised in that** the self-cutting outer thread (4) is outwardly pointed.

4. An artificial acetabular cup according to claim 1, **characterised in that** the core of the self-cutting outer thread (4) projects outwardly over the intended extended spherical surface at the beginning and at the end of outer thread (4), and falls below in the central area.

5. An artificial acetabular cup according to claim 4, **characterised in that** the points of outer thread (4) are located in a spherical surface which is concentric to the part-spherical shaped section (2).

## Revendications

1. Cotyle artificiel de la hanche, avec une surface extérieure spatialement arquée qui présente une section (2) antérieure partiellement sphérique et une section (3) postérieure présentant un filet extérieur (4) autotaraudeur s'étendant depuis un noyau conique, le filet extérieur (4) faisant saillie vers l'extérieur de la surface sphérique imaginaire de prolongement de la section partiellement sphérique et les flancs du filet extérieur (4) ayant, dans la région du noyau, un écartement l'un de l'autre,
**caractérisé en ce que** le noyau conique portant le filet extérieur (4) autotaraudeur fait saillie au moins partiellement vers l'extérieur par rapport à la surface sphérique imaginaire de prolongement de la section partiellement sphérique,
que des arêtes de coupe (6) sont formées sur la surface extérieure du noyau à filet et
que les évidements (5) que forment les arêtes de coupe (6) du filet extérieur (4) autotaraudeur s'étendent au moins jusqu'à la surface sphérique imaginaire prolongée.

2. Cotyle artificiel de la hanche selon la revendication 1,
**caractérisé en ce que** les arêtes de coupe (6) sont formées sur des saillies à la surface extérieure libre du noyau.

3. Cotyle artificiel de la hanche selon la revendication 1,
**caractérisé en ce que** le filet extérieur (4) autotaraudeur est tranchant en direction de l'extérieur.

4. Cotyle artificiel de la hanche selon la revendication 1,
**caractérisé en ce que** le noyau du filet extérieur (4) autotaraudeur, par rapport à la surface sphérique imaginaire prolongée, fait saillie vers l'extérieur au début et à la fin du filet extérieur (4) et est en-dessous dans la zone médiane.

5. Cotyle artificiel de la hanche selon la revendication 4,
**caractérisé en ce que** les tranchants du filet extérieur (4) se trouvent dans une surface sphérique qui est concentrique à la section (2) partiellement sphérique.
